# EUROPEAN PATENT APPLICATION

(11) **EP 1 016 423 A1**
(43) Date of publication of application: **05.07.2000**
(21) Application number: 99310490.0
(22) Date of filing: 23.12.1999
(51) Int. Cl.: A61L 29/06, A61L 31/06, A61L 27/18, C08G 18/38

(54) **Radiopaque polymer blend**

(30) Priority: 31.12.1998 US 224444
(71) Applicant: ETHICON INC., Somerville New Jersey 08876 (US)
(72) Inventor: Onwumere, Fidelis C., Mansfield, Texas 76063 (US)
(74) Representative: Mercer, Christopher Paul

(57) **Abstract**

A medical implant such as a catheter, stent, vascular graft or other implant, comprised of a blend of two polymers. In one embodiment, the first polymer includes a diisocyanate, a polyol, and a first chain extender having a bromine concentration greater than about 30 percent. The second polymer comprises a diisocyanate, a polyol, and a second chain extender having a bromine concentration less than about 10 percent.

## Description

### BACKGROUND OF THE INVENTION

### Field of the Invention

The invention relates generally to medical implants and more particularly to medical implants including tubing for use in catheters, stents, and other devices.

### Background of the Invention

In certain medical procedures, medical implants are placed into the body. These implants include catheters inserted into body passages, vessels, or cavities for passing fluids, draining fluids, making examinations, etc. A stent is a second type of medical implant used to maintain an orifice or cavity during skin grafting or to provide support for the lumen of tubular structures, for example, during or after an anastomosis.

It is generally desirable that medical implants, such as catheters and stents be radiographically opaque such that their precise location within the host body by X-ray examination can be detected. In addition, it is advantageous that such medical implants be optically transparent so that a flow of fluid therethrough may be observed.

Many tubular-shaped medical implants, such as catheters and stents include tubing that is made from a polymer base. The polymers are chosen such that they can be formed into tubular shapes that are, particularly in the case of catheters, flexible enough to be routed or snaked, to a location in the body. In the case of a peripherally inserted central catheter, for example, the tubing of the catheter is routed or snaked, in one instance, through a vein in a patient's arm or neck to the superior vena cava of the patient's heart. The tubing must be flexible enough to be routed in this manner without causing trauma to the patient. The polymer chosen as the medical implant should also have sufficient strength when formed into tubing so that the tubing does not collapse in a passageway or orifice. Still further, the tubing should be resistant to crimping or kinking so that a continuous passageway is assured.

Polyurethane-based polymers are a popular choice for medical implant polymers. In general, thermoplastic polyurethanes are condensation products of reactions between diisocyanate (isocyanate compounds having a functionality of two) and soft-block polyols.

Typically, polyurethanes of diisocyanates and polyols are combined with low molecular weight aliphatic diols, and aliphatic diols having aromatic residues as "chain extenders" to impart the useful properties of flexibility, strength, and kink resistance. Low molecular weight diols include butane diol, pentane diol, hexane diol, heptane diol, benzene dimethanol, hydroquinone diethanol, and ethylene glycol. The diisocyanates may alternatively be combined with diamines, such as ethylene diamine, butane diamine, propane diamine, etc., to form a class of polyurethanes known as polyurethaneureas. An added feature of these polyurethanes, including polyurethaneureas, is that catheter or stent tubing formed from these materials is typically optically transparent making these polymer matrices excellent compounds for medical implants. Unfortunately, however, these polyurethanes are not radiopaque.

Radiopaque medical implants such as catheters, including radiopaque polyurethanes, have been developed. These radiopaque polymer structures are generally of two forms. A first form of radiopaque polymer incorporates a filler such as barium sulfate (BaSO₄), bismuth subcarbonate, or certain metals such as tungsten (W). The filler provides the polymer with its radiopacity. Unfortunately, the filler also tends to make the polymer non-transparent.

A second form of radiopaque polymer useful in medical implants such as catheters incorporates a halogenated-chain extender into the polymer matrix. Examples of these types of polymers are described in U.S. Patent Nos. 4,722,344; 5,177,170; and 5,346,981. The preferred halogen in these patents is bromine (Br). Polymers incorporating a brominated-chain extender into the polymer matrix generally yield a tubing that is radiopaque and optically transparent.

In order to impart useful radiopaque properties, the halogenated-chain extended polymer, such as a bromine-chain extended polymer, must have a minimum amount of halogen (e.g., bromine) to impart radiopacity to the polymer. Experimental studies show that the minimum amount of bromine, for example, in a polyurethane-based polymer useful as catheter tubing, is approximately 15 percent. Amounts less than this tend to make the tubing less detectable by X-ray.

A second problem with halogenated-chain extended polymers is the maximum amount of halogen that can be incorporated into the polymer is limited. Experimental studies have shown that polymers having, for example, a bromine concentration greater than 30 percent are too stiff for use as a medical implant, such as a catheter tubing. Accordingly, the radiopacity of the tubing is limited by the amount of bromine or other halogen that may be incorporated in the polymer matrix without degrading the properties of the tubing made from such a polymer.

As noted above, certain halogenated-chain extended polymers offer both radiopacity and optical transparency. However, in order to maintain the superior properties demonstrated by conventional thermoplastic polyurethane with non-halogenated chain extenders, the amount of halogen must be strictly limited. It would be desirable, in certain instances, to have a halogenated-chain extended polymer with a radiopaque property that is not limited by the amount of halogen that is incorporated into the polymer matrix. What is needed is a halogenated polymer, such as a halogenated polyurethane, that can maximize the amount of halogenated chain extender without negatively affecting the physical characteristics of the medical implant.

### SUMMARY OF THE INVENTION

A medical implant such as a catheter, stent, vascular graft, or other implant is disclosed. In one embodiment, the implant is comprised of a combination of two polymers. The first polymer includes a diisocyanate, a polyol, and a first chain extender having a bromine concentration greater than about 30 percent by weight of the first polymer. The second polymer has a bromine concentration less than about 10 percent. According to the invention, a suitable medical implant can be formed by combining the bromine-chain extended polymer with a variety of other polymers to incorporate useful properties, including transparency and radiopacity, into the composition.

In another embodiment, the first polymer and the second polymer are diisocyanate-based polyurethanes blended in an amount such that the overall bromine concentration of the medical implant lies within the range of approximately 15-30 percent and, preferably, 15-24 percent. This may be achieved by combining, for example, a first polymer having a bromine concentration greater than 30 percent and a second polymer having a negligible bromine concentration in a ratio of about 70 percent first polymer to about 30 percent second polymer.

In one aspect, the invention seeks to utilize the beneficial elastomeric properties of a non-halogenated-chain extended polymer, including flexibility, kink resistance, etc., with the added property of consistently detectable levels of bromine to make the composition radiopaque. By combining polymers in the manner described in the invention to form a radiopaque polymer, the implant of the invention is not restricted to the physical, chemical, and mechanical properties achieved by singly using, for example, a bromine-chain extended polyurethane as in the prior art.

Additional features, embodiments, and benefits will become apparent from the figures, detailed description, and claims set forth below.

### BRIEF DESCRIPTION OF THE DRAWINGS

The features, aspects, and advantages of the invention will become more thoroughly apparent from the following detailed description, appended claims, and accompanying drawings in which:
**Figure 1** is a flow chart of a blending process in making a medical implant according to an embodiment of the invention.
**Figure 2** is a flow chart of a co-extrusion process in making a medical implant according to an embodiment of the invention.

### DETAILED DESCRIPTION OF THE INVENTION

The invention relates to a medical implant, including the tubing or cannula of a catheter, stent, vascular graft, or similar device. The medical implant is comprised of a first polymer and a second polymer combined such as by an extrusion or co-extrusion method. In one embodiment, the first polymer is a halogenated-chain extended polyurethane. In this manner, the first polymer comprises a diisocyanate, a polyol, and a first chain extender having, for example, a bromine concentration greater than about 30 percent. The second polymer has a halogen (e.g., bromine) concentration less than about 10 percent. In one embodiment, the second polymer is also a polyurethane-based polymer of, for example, a diisocyanate, a polyol, and a second chain extender having a chemically insignificant amount of bromine. It is to be appreciated that other polymers that add useful properties may be substituted for the polyurethane base of either the first or second polymer or combined with the first or second polymer as will become evident from the description that follows.

In one embodiment, the first polymer is a polyurethane comprising a diisocyanate, a polyol, and a chain extender having a bromine concentration greater than about 30 percent. In prior art compositions, the bromine concentration in the polymer was typically less than about 30 percent by weight of the polymer to maintain suitable physical properties. The first polymer of the invention, however, utilizes a chain extender having a bromine concentration greater than about 30 percent by weight of the first polymer. The invention contemplates that the first polymer may contain up to the stoichiometrically allowable amount of bromine. The brominated chain extender is preferably used in an amount that will maximize the radiopacity of the composition. Suitable bromine-chain extenders according to the invention include, but are not limited to, brominated bisphenol-A-diethanol, brominated hydroquinone diethanol, brominated benzene dimethanol, brominated biphenyl oxydiethanol, and other aromatic brominated chain extenders, preferably with a population of approximately four bromine atoms per molecule on the aromatic ring.

The first polymer that is, for example, a polyurethane is prepared by any of the several methods known in the art, including but not limited to the "one-shot" process, the "prepolymer" process, and the "semi-prepolymer" process. One example of a preparation process is the "one-shot" process where the isocyanate-reactive components, including the polyol and the halogenated-chain extender, as well as any additives, including optional catalysts are combined into a premix. Suitable catalysts include, but are not limited to, organic metal compounds, such as organic tin compounds (dioctyl tin mercaptide, tin (II) acetate, etc.) and teritiary amines such as triethylamine. The diisocyanate compound is then combined with the premix to make the first polymer. A solid composition is formed that is then ground and pelletized according to known techniques.

In one embodiment, the second polymer is selected based primarily on its physical and chemical properties. In the case where the second polymer is a polyurethane, for example, the second polymer includes preferably a diisocyanate, a polyol, and a chain extender. The chain extender is, for example, a low molecular weight diol including, but not limited to, ethylene glycol, propylene glycol, butane diol, pentane diol, hexane diol, heptane diol, and benzene dimethanol, hydroquinone diethanol, and isomers of the same. A chain extender that is a diamine, such as ethylene diamine, propane diamine, etc., may also be utilized.

A polyurethane such as described for the second polymer is generally optically transparent. The polyurethane has superior physical properties, including flexibility or flexural strength, and a resistance to kinking. In one embodiment, the second polymer, such as a polyurethane as described, also includes a small amount of a halogenated-chain extender. By keeping the amount of halogenated-chain extender less than about 10 percent, the physical properties noted are generally not effected.

### Example 1

The following represents one example of a composition of a medical implant such as catheter tubing according to an embodiment of the invention.

| Polymer A (100 g sample) | Amount |
|---|---|
| Polytetramethylene ether glycol (PTMEG) commercially available from E.I. duPont de Nemours & Co. of Wilminton, Delaware | 11.4 g |
| Tetrabromobis-phenol-A-diethanol (TBA-2) commercially available from Goulston Technologies of Monroe, North Carolina | 62.2 g |
| Diphenylmethane diisocyanate (MDI) commercially available from Bayer International Chemical Division of Pittsburgh, Pennsylvania | 26.4 g |

The first polymer contains 31.50% bromine by weight.

| |
|---|
| Polymer B |
| PELLETHANE™, a thermoplastic polyurethane elastomer commercially available from E.I. duPont de Nemours & Co. of Wilmington, Delaware. (PELLETHANE™ does not contain bromine.) |

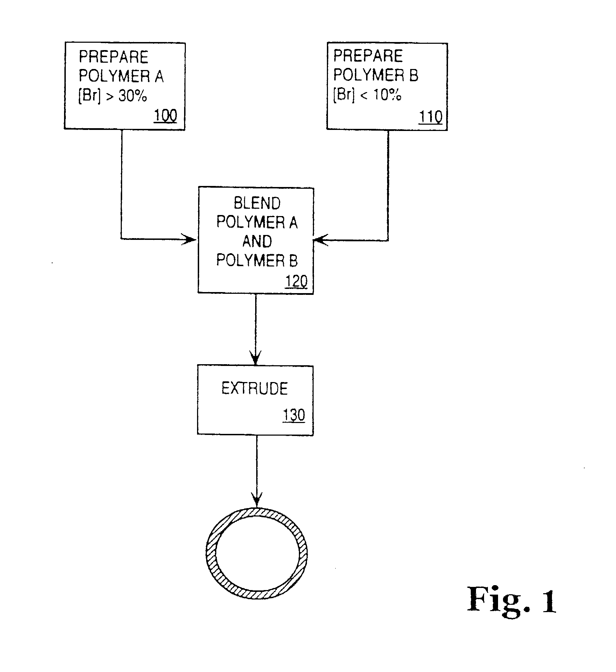

In one embodiment, the composition of the invention is prepared by combining the first polymer with the second polymer. **Figure 1** shows a flowchart of one process of the invention. According to **Figure 1**, in step 100, a first polymer, such as the polyurethane described in the above example as polymer A (i.e., a polyurethane of diisocyanate, a polyol, and a chain extender having a bromine concentration greater than 30 percent of the weight of the polymer) is prepared according to known techniques. In step 110, a second polymer such as the PELLETHANE™ polymers described in the above example as polymer B having a bromine concentration less than about 10 percent is also prepared. These polymers are combined in step 120, such as for example, in a thermal extrusion apparatus, and thermally extruded in step 130 into a single apparatus such as a medical implant including, but not limited to, a catheter tubing or other implantable device that may be detected by X-ray.

### Example 2

The following represents various blending ratios of the first and second polymers of Example 1 according to the process described with reference to **Figure 1** and the accompanying text.

| Formulation | First Polymer | Second Polymer | Wt% Bromine |
|---|---|---|---|
| 1 | 70 | 30 | 22 |
| 2 | 60 | 40 | 19 |
| 3 | 50 | 50 | 15.8 |
| 4 | 48 | 52 | 15.2 |

Experimental studies have shown that the elastomeric property, particularly the kink resistance, is improved for Formulations 2, 3, 4 above, in particular, then a medical implant made of the first polymer only.

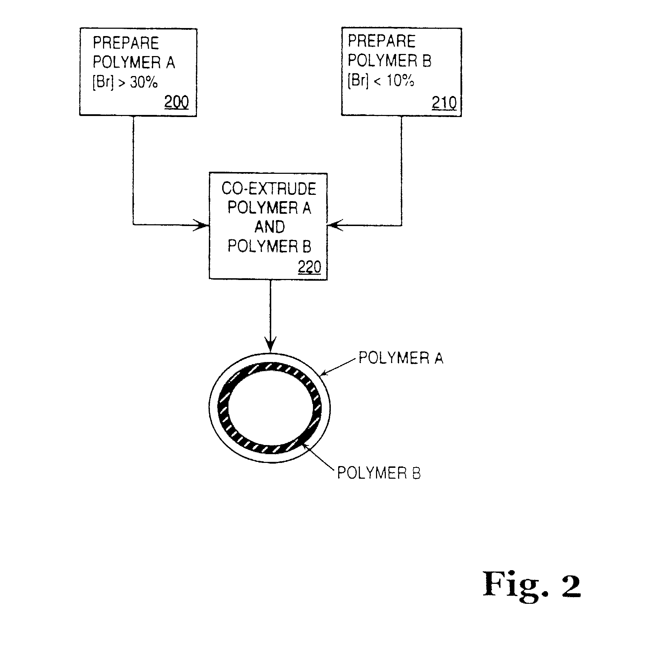

**Figure 2** shows a schematic of a co-extrusion process to form a medical implant according to a second embodiment of the invention. In the co-extrusion process of **Figure 2**, a first polymer, such as the polymer A described in Example 1, (step 200) is extruded along with a similar extrusion of the second polymer such as the polymer B described in Example 2, (step 210) in a conventional co-extrusion process (step 220). The resulting medical implant is a layered structure, such as the layered tubing shown in **Figure 2**. It is to be appreciated that the location of polymer A as the outer layer is optional and the desired co-extrusion process may reverse the layers.

The preceding detailed description focused on the combination of two polymers. It is to be appreciated that additional polymers or additives, for that matter, may be combined with the two polymers to, in certain instances, further enhance the properties of the ultimate composition including a medical implant. For example, the polyurethane can be blended with other medical grade polymers such as polyether amide, polyether ester, and non-urethane-based thermoplastic elastomers.

In the preceding detailed description, the invention is described with reference to specific embodiments thereof. It will, however, be evident that various modifications and changes may be made thereto without departing from the broader spirit and scope of the invention as set forth in the claims. The specification and drawings are, accordingly, to be regarded in an illustrative rather than a restrictive sense.

## Claims

1. A medical implant or tubing comprising (a) a first polymer comprising: a diisocyanate; a polyol; and a first chain extender having a bromine concentration greater than about 30 percent by weight of the first polymer and (b) a second polymer having a bromine concentration less than about 10 percent.

2. The medical implant or tubing of claim 1, wherein the first chain extender comprises a brominated-chain extender.

3. The medical implant or tubing of claim 1 or claim 2, wherein the first chain extender is brominated bisphenol-A-diethanol.

4. The medical implant or tubing of any one of claims 1 to 3, wherein the second polymer comprises a chemically insignificant amount of bromine.

5. The medical implant or tubing of any one of claims 1 to 4, wherein the second polymer comprises: a diisocyanate; a polyol; and a second chain extender.

6. The medical implant or tubing of claim 5, wherein the second chain extender is an alkyl diol, ethylene glycol or phenyl dimethanol.

7. The medical implant or tubing of any one of claims 1 to 6, comprising about 70 percent first polymer and about 30 percent second polymer.

8. The medical implant or tubing of any one of claims 1 to 6, further comprising a third polymer that is a polycarbonate, a polyurethaneurea or a polyamide.
